# EUROPÄISCHE PATENTSCHRIFT

(11) **EP 0 601 361 B1**
(45) Veröffentlichungstag und Bekanntmachung des Hinweises auf die Patenterteilung: **04.11.1998**
(21) Anmeldenummer: 93118645.6
(22) Anmeldetag: 19.11.1993
(51) Int. Cl.: C09B 62/09, D06P 1/382

(54) **Wasserlösliche Disazoverbindungen, Vefahren zu ihrer Herstellung und ihre Verwendung als Farbstoffe**
Water-soluble disazo compounds, process for their preparation and their use as dyestuffs
Composés disazoiques solubles dans l'eau, procédé pour leur préparation et leur utilisation comme colorants

(30) Priorität: 25.11.1992 DE 4239519
(43) Veröffentlichungstag der Anmeldung: 15.06.1994
(73) Patentinhaber: DyStar Textilfarben GmbH & Co. Deutschland KG, 60318 Frankfurt am Main (DE)
(72) Erfinder: Von der Eltz, Andreas, Dr., D-60433 Frankfurt am Main (DE); Russ, Werner Hubert, Dr., D-65439 Flörsheim/Main (DE)

(56) Entgegenhaltungen:
- EP-A- 0 083 052
- EP-A- 0 356 681
- CH-A- 471 870
- FR-A- 2 335 570
- FR-A- 2 398 095
- PATENT ABSTRACTS OF JAPAN, Band 11, Nr. 30 (C-400)(2477), 20 Jänner 1987

## Beschreibung

Die Erfindung liegt auf dem technischen Gebiet der faserreaktiven Farbstoffe.

Aus den U.S.-Patentschriften 4 329 282 und 4 798 887 und der britischen Patentschrift 1 102 204 sind Phenylazonaphthyl-azophenylamino- und Phenylazonaphthyl-azonaphthylamino-Verbindungen bekannt, an deren Aminogruppe ein faserreaktiver Halogen-triazinyl-Rest gebunden ist, die als Farbstoffe Färbungen mit brauner Nuance liefern. Insbesondere ist aus dem Tabellenbeispiel 47 der erwähnten US-PS 4 329 282 eine Disazoverbindung bekannt, die den 4-Carboxy-phenylamino-Rest als Substituenten an den Fluortriazin-Rest enthält; diese Disazoverbindung besitzt jedoch ausgesprochen mangelhafte färberische Eigenschaften.

Darüberhinaus sind aus der japanischen Patentanmeldungs-Veröffentlichung Sho-61-200174 (referiert in Patent Abstracts of Japan, Vol. 11, No. 30 (1987)) Disazofarbstoffe mit einer endständigen Naphthol-Kupplungskomponente beschrieben, wobei diese naphtholische Kupplungskomponente einen Fluortriazinrest enthält, der durch einen Phenylamino- oder Naphthylamino-Substituenten substituiert ist, dessen aromatischer Rest durch unterschiedlichste Substituenten substituiert sein kann, unter anderem auch durch eine Carboxygruppe.

Mit der vorliegenden Erfindung wurden nunmehr neue Disazoverbindungen der nachstehend angegebenen und definierten allgemeinen Formel (1) gefunden, die sehr gute Farbstoffeigenschaften mit verbesserten anwendungstechnischen Eigenschaften besitzen und auf hydroxy- und/oder carbonamidgruppenhaltigen Fasermaterialien Färbungen mit guten Echtheitseigenschaften und einer sehr guten Auswaschbarkeit nicht fixierter Farbstoffanteile liefern. 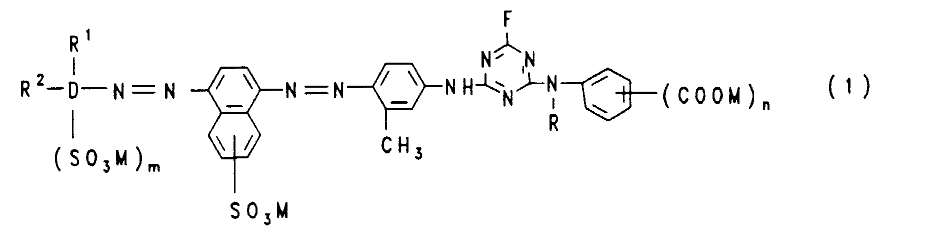 In Formel (1) bedeuten:
- D: ist ein Phenyl- oder Naphthylrest, wobei der Naphthylrest bevorzugt ein Naphth-2-yl-Rest ist;
- R¹: ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Carboxy, Halogen, wie Chlor, Brom und Fluor, Alkanoylamino von 2 bis 5 C-Atomen, wie Acetylamino und Propionylamino, oder Benzoylamino, bevorzugt Wasserstoff;
- R²: ist Wasserstoff oder Alkyl von 1 bis 4 C-Atomen, wie Ethyl und insbesondere Methyl, bevorzugt Wasserstoff;
- M: ist Wasserstoff oder ein Alkalimetall, wie Natrium, Kalium und Lithium, oder ein anderes salzbildendes Metall;
- m: ist die Zahl 1, 2 oder 3, bevorzugt 2 oder 3;
- n: ist die Zahl 1, wobei die Gruppe -COOM in ortho-Stellung zur Gruppe -N(R)- an den Benzolkern gebunden ist ;
- R: ist Wasserstoff oder Alkyl von 1 bis 4 C-Atomen, wie Methyl und Ethyl, bevorzugt Wasserstoff;
die Gruppe SO₃M an der Naphthalin-Mittelkomponente sitzt in 6-, 7- oder 8-Stellung, bevorzugt in 6- oder 7-Stellung, an diesen Naphth-4,1-ylen-Rest gebunden.

Bevorzugt von den Verbindungen der allgemeinen Formel (1) sind solche, in welchen D für den 2-Naphthyl-Rest steht, R¹ und R² beide Wasserstoff bedeuten und m die Zahl 2 oder 3 ist. Sofern D ein Phenylrest ist, haben R¹ und R² die oben genannten Bedeutungen und m steht bevorzugt für die Zahl 1 und insbesondere bevorzugt die Zahl 2, wobei R¹ bevorzugt Wasserstoff, Methyl, Methoxy, Carboxy, Chlor, Brom, Acetylamino oder Benzoylamino und R² bevorzugt Wasserstoff oder Methyl ist und R¹ und R² beide insbesondere bevorzugt Wasserstoff bedeuten.

Die Gruppen "Sulfo" und "Carboxy" schließen sowohl deren Säureform als auch deren Salzform ein. Demgemäß bedeuten Sulfogruppen Gruppen entsprechend der allgemeinen Formel -SO₃M und Carboxygruppen Gruppen entsprechend der allgemeinen Formel -COOM mit M der oben genannten Bedeutung.

Die vorliegende Erfindung betrifft weiterhin Verfahren zur Herstellung der erfindungsgemäßen Disazoverbindungen. Sie sind dadurch gekennzeichnet, daß man eine Amino-Disazoverbindung der allgemeinen Formel (2) 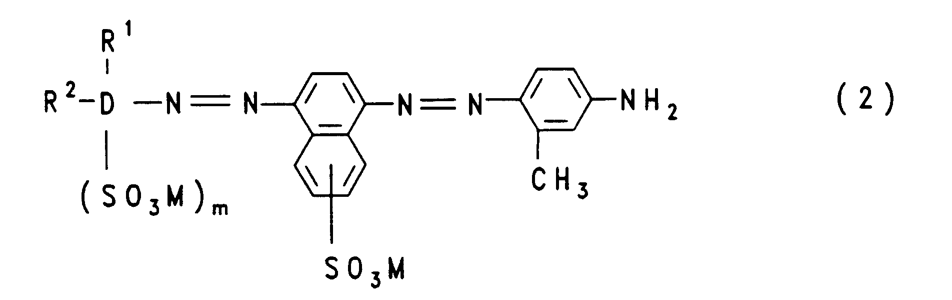 in welcher D, R¹, R², M und m eine der oben genannten Bedeutungen besitzen, mit Cyanurfluorid (2,4,6-Trifluor-1 3,5-triazin) umsetzt und die so erhaltene Difluor-triazinylamino-Disazoverbindung der allgemeinen Formel (3) 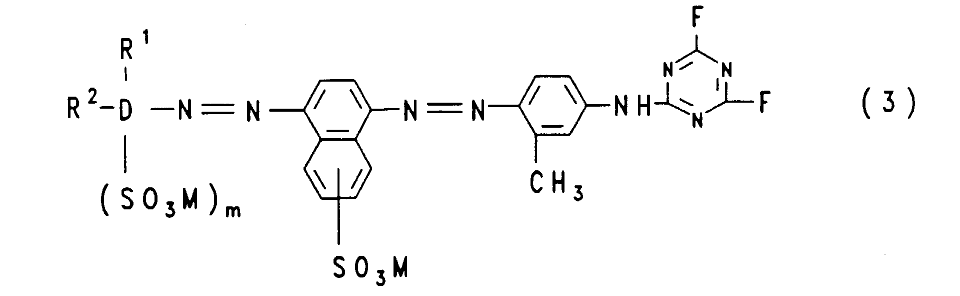 in welcher D, R¹, R², M und m eine der oben genannten Bedeutungen besitzen, mit einer Verbindung der allgemeinen Formel (4) 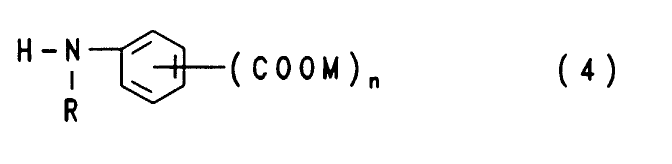 in welcher R, M und n eine der oben genannten Bedeutungen haben, umsetzt, oder daß man eine Verbindung der allgemeinen Formel (2) mit einer Verbindung der allgemeinen Formel (5) 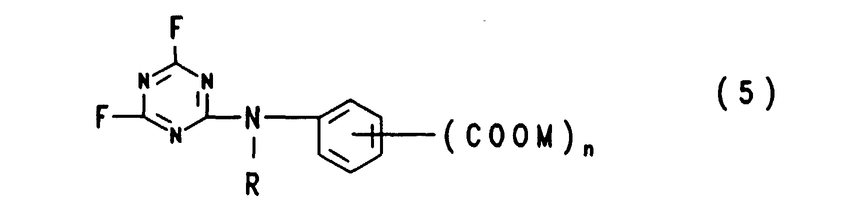 in welcher R, M und n eine der oben genannten Bedeutungen haben, umsetzt.

Die erfindungsgemäße Verfahrensvariante der Umsetzung einer Verbindung der allgemeinen Formel (2) mit Cyanurfluorid und anschließend mit einer Verbindung der allgemeinen Formel (4) kann in wäßrigem oder in wäßrig-organischem Medium durchgeführt werden; bevorzugt erfolgt sie in wäßriger Lösung bzw. Suspension. Bei Verwendung eines wäßrig-organischen Reaktionsmediums ist der organische Lösemittelanteil ein gegenüber den Reaktanten inertes Lösemittel, wie beispielsweise Aceton, Dimethylformamid, Dioxan oder Dimethylsulfoxid. Die Umsetzung der Amino-Disazoverbindung der allgemeinen Formel (2) mit Cyanurfluorid erfolgt in der Regel bei einer Temperatur zwischen -10°C und +30°C, vorzugsweise zwischen + 5°C und + 15°C, und bei einem pH-Wert zwischen 6 und 8, bevorzugt zwischen 4 und 6.
Die anschließende Umsetzung der Difluortriazinylamino-Disazoverbindung der allgemeinen Formel (3) mit der Verbindung der allgemeinen Formel (4) erfolgt in der Regel, bevorzugt ohne Isolierung der zuvor hergestellten Verbindung (3) aus dem Reaktionsansatz, bei einer Temperatur zwischen 5 und 20°C, bevorzugt zwischen 5 und 10°C, und bei einem pH-Wert zwischen 5 und 8, insbesondere zwischen 6 und 7,5.

Die erfindungsgemäße Umsetzung einer Verbindung der allgemeinen Formel (2) mit einer Verbindung der allgemeinen Formel (5) kann ebenfalls in wäßrigorganischem Medium erfolgen; bevorzugt wird sie in wäßrigem Medium durchgeführt. In der Regel erfolgt sie bei einer Temperatur zwischen -5°C und + 20°C, insbesondere zwischen 0 und 10°C, und bei einem pH-Wert zwischen 6 und 9, insbesondere 6,5 und 7,5.

Die Ausgangsverbindungen der allgemeinen Formel (2) werden in an und für sich bekannter und üblicher Weise durch Diazotierung und Kupplung von deren entsprechende Ausgangs-Komponenten hergestellt, so durch Kupplungsreaktion von 6-, 7- oder 8-Sulfo-1-aminonaphthalin mit der Diazoverbindung des aromatischen Amins der allgemeinen Formel (6) 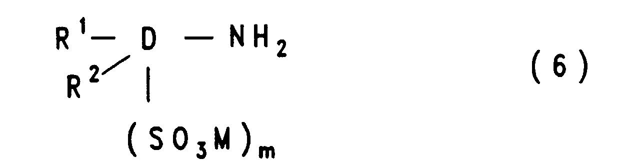 in welcher R¹ , R², D, M und m eine oder oben genannten Bedeutungen haben, und anschließende Diazotierung der so erhaltenen Amino-Monoazoverbindung und Kupplung mit 3-Methyl-anilin.
Die Ausgangsverbindungen der allgemeinen Formel (5) können hergestellt werden, indem man Cyanurfluorid mit einer Verbindung der allgemeinen Formel (4) in wäßrig-organischem Medium (wobei der organische Lösemittelanteil wie oben angegeben ist) oder wäßrigem Medium bei einer Temperatur zwischen 0 und 10°C und bei einem pH-Wert zwischen 0 und 1 unter Zugabe einer äquimolaren Menge von Natriumfluorid oder bei einem pH-Wert zwischen 5 und 7,5 in einem Fließsystem unter Ausschluß von Rückvermischung umsetzt.

Ausgangsverbindungen der allgemeinen Formel (6) sind beispielsweise 2-Sulfo-4-methoxy-anilin, 2-Sulfo-4-ethoxy-anilin, 2,5-Disulfo-4-methoxy-anilin, 2,5-Disulfo-4-ethoxy-anilin, 2,5-Disulfo-anilin, 2,6-Dimethyl-3,5-disulfo-anilin, 4,8-Disulfo-2-amino-naphthalin, 1,5-Disulfo-2-amino-naphthalin, 6,8-Disulfo-2-amino-napthalin, 3,6,8-Trisulfo-2-amino-naphthalin und 4,6,8-Trisulfo-2-amino-naphthalin.

Die Abscheidung und Isolierung der erfindungsgemäß hergestellten Disazoverbindungen der allgemeinen Formel (1) aus den Syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei der Syntheselösung eine Puffersubstanz zugefügt werden kann.

Die erfindungsgemäßen Disazoverbindungen der allgemeinen Formel (1) - im nachfolgenden Verbindungen (1) genannt - haben faserreaktive Farbstoffeigenschaften. Sie können deshalb zum Färben (einschließlich Bedrucken) von hydroxygruppenhaltigen und/oder carbonamidgruppenhaltigen Fasermaterialien verwendet werden. Auch können die bei der Synthese der Verbindungen (1) anfallenden Lösungen, gegebenenfalls nach Zusatz einer Puffersubstanz und gegebenenfalls auch nach Konzentrierung, direkt als Flüssigpräparation der färberischen Verwendung zugeführt werden.

Gegenstand der vorliegenden Erfindung ist deshalb auch die Verwendung der erfindungsgemäßen Verbindungen (1) zum Färben (einschließlich Bedrucken) von hydroxy- und/oder carbonamidgruppenhaltigen Fasermaterialien bzw. Verfahren zum Färben von solchen Substraten unter Verwendung einer Verbindung (1) als Farbstoff. Bevorzugt werden die Materialien in Form von Textilfasern, wie Garnen, Wickelkörpern und Geweben eingesetzt.

Hydroxygruppenhaltige Materialien sind solche natürlichen oder synthetischen Ursprungs, wie beispielsweise Cellulosefsermaterialien und deren Regeneratprodukte oder Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten nach den für wasserlöslichen Farbstoffe, insbesondere faserreaktive Farbstoffe, bekannten Anwendungstechniken applizieren und fixieren, so beispielsweise, indem man die Verbindung (1) in gelöster Form auf das Substrat aufbringt oder sie darin einbringt und sie auf oder in dem Substrat durch Hitzeeinwirkung oder durch Einwirkung eines alkalisch wirkenden Mittels oder durch beide Maßnahmen fixiert. Solche Färbe- und Fixierweisen sind sowohl in der Fachliteratur als auch in der Patentliteratur zahlreich beschrieben, wie beispielsweise in den anfangs genannten Druckschriften.

Mit den Verbindungen (1) werden sowohl auf carbonamidgruppenhaltigen Materialien, wie insbesondere auf Wolle, als auch auf hydroxygruppenhaltigem Material, wie insbesondere Cellulosefasermaterial, braune, insbesondere rotstichig braune, Färbungen und Drucke mit hoher Farbausbeute und gutem Farbaufbau bei sehr geringer Temperatur- und Alkaliabhängigkeit erhalten. Wegen ihres besonderen rotstichigen Brauntons können sie mit besonderem Vorteil in der Trichromiefärbung Verwendung finden. Die mit den erfindungsgemäßen Verbindungen (1) erhältlichen Färbungen und Drucke besitzen gute Echtheitseigenschaften, wie gute Licht- und Naßechtheitseigenschaften, so beispielsweise eine gute Trockenlichtechtheit und gute Naßlichtechtheiten des mit Trinkwasser oder einer sauren oder alkalischen Schweißlösung befeuchteten gefärbten Materials, gute Waschechtheiten bei 60 bis 95°C, auch in Gegenwart von Perboraten, saure und alkalische Walk-, Überfärbe- und Schweißechtheiten, gute Alkali-, Säure-, Wasser- und Seewasserechtheiten und gute Chlorwasser- und Hypochloritechtheiten, des weiteren eine gute Plissierechtheit, Bügelechtheit und Reibechtheit.

Von den Färbungen und Drucken auf carbonamidgruppenhaltigem Material, wie insbesondere auf Wolle, können die Licht-, Wasch- und Naßlichtechtheiten hervorgehoben werden, selbst wenn auf eine ansonsten übliche ammoniakalische Nachbehandlung der gefärbten Ware verzichtet wird. Ebenso läßt sich Material aus Wollfasern unterschiedlicher Provenienz mit den erfindungsgemäßen Azoverbindungen egal färben, wobei gegebenenfalls zur Verbesserung des Egalisierverhaltens ein übliches Egalisierhilfsmittel, wie beispielsweise N-Methyl-taurin zugesetzt werden kann.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säuren angegeben; im allgemeinen werden sie in Form ihrer Alkalimetallsalze hergestellt und isoliert und in Form ihrer Salze zum Färben verwendet. Ebenso können die in den nachfolgenden Beispielen in Form der freien Säure genannten Ausgangsverbindungen und Komponenten als solche oder in Form ihrer Salze, vorzugsweise Alkalimetallsalze, in die Synthese eingesetzt werden.
Die im sichtbaren Bereich angegebenen Absorptionsmaxima (λₘₐₓ-Werte) wurden aus wäßriger Lösung der Alkalimetallsalze bestimmt.

### Beispiel 1

303 Teile 2-Aminonaphthalin-4,8-disulfonsäure werden in 1000 Teilen einer schwefelsauren wäßrigen Lösung in üblicher Weise durch Zugabe einer 40 %igen wäßrigen Natriumnitritlösung diazotiert. Man gibt sodann 223 Teile 1-Amino-naphthalin-6-sulfonsäure hinzu und führt die Kupplungsreaktion bei einem pH-Wert zwischen 4 und 5 und einer Temperatur zwischen 10 und 15°C durch. Diesen Ansatz stellt man sodann mit Schwefelsäure auf einen pH-Wert unterhalb von 2 und diazotiert in gleicher Weise mittels Natriumnitrit (in jedem Falle wird nach der Diazotierungsreaktion überschüssige salpetrige Säure in üblicher Weise mittels Amidosulfonsäure zerstört), gibt eine Lösung von 108 Teilen 3-Methyl-anilin in 100 Teilen einer 33 %igen wäßrigen Salzsäure hinzu, stellt den pH-Wert mittels Natriumcarbonat auf 3,5 bis 4, führt die Kupplungsreaktion innerhalb dieses pH-Bereiches bei einer Temperatur von etwa 20°C durch, stellt anschließend den pH-Wert auf 7, kühlt den Ansatz auf 5°C ab und gibt langsam unter Einhaltung eines pH-Bereiches zwischen 6 und 7 und einer Temperatur zwischen 0 und 10°C 135 Teile Cyanurfluorid hinzu. Danach setzt man 139 Teile Anthranilsäure hinzu und führt die zweite Kondensationsreaktion bei einer Temperatur zwischen 5 und 10°C und einem pH-Wert zwischen 6 und 7,5 durch.

Der erhaltene Syntheseansatz wird mittels 20 Teilen Kieselgur und Filtration geklärt. Die erfindungsgemäße Disazoverbindung der (in Form der freien Säure geschrieben) Formel 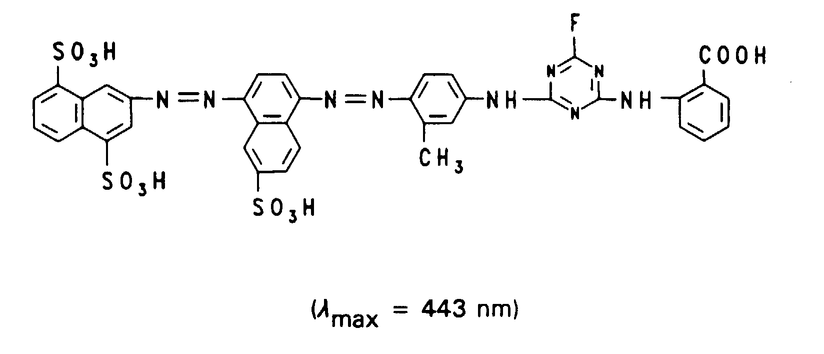 wird durch Eindampfen oder Sprühtrocknung des Filtrats oder durch Aussalzen mittels Natriumchlorid als Alkalimetallsalz (Natriumsalz) isoliert.

Diese Disazoverbindung besitzt sehr gute Farbstoffeigenschaften und liefert auf den in der Beschreibung genannten Materialien, insbesondere Cellulosefasermaterialien, wie Baumwolle, nach den in der Technik für faserreaktive Farbstoffe üblichen Applikations- und Fixiermethoden farbstarke, rotstichig braune Färbungen und Drucke mit guten Echtheitseigenschaften, von denen insbesondere die hohe Lichtechtheit und Hypochloritechtheit hervorgehoben werden können.

### Beispiel 2

Zur Herstellung einer erfindungsgemäßen Disazoverbindung stellt man zunächst gemäß der Verfahrensweise des Beispieles 1 die Amino-disazo-Ausgangsverbindung durch Kupplung des Diazoniumsalzes aus 383 Teilen 2-Amino-naphthalin-4₁6,8-trisulfonsäure mit 223 Teilen 1-Amino-naphthalin-6-sulfonsäure und anschließende Diazotierung der erhaltenen Amino-Monoazoverbindung und Kupplung mit 108 Teilen 3-Methyl-anilin her. Der die Amino-Disazoverbindung enthaltende Reaktionsansatz wird sodann bei einer Temperatur zwischen 0°C und 5°C und unter Einhaltung eines pH-Wertes von 7 mittels Natriumcarbonat mit dem Reaktionsprodukt aus 135 Teilen Cyanurfluorid, 139 Teilen Anthranilsäure, 84 Teilen Natriumfluorid und 100 Teilen einer 33%igen wäßrigen Salzsäure versetzt. Der Ansatz wird bei etwa 20°C und unter Einhaltung eines pH-Wertes von 7 noch etwa 60 Minuten nachgerührt und anschließend durch Zugabe von 20 Teilen Kieselgur und durch Filtration geklärt.

Die erhaltene erfindungsgemäße Disazoverbindung der (in Form der freien Säure geschrieben) Formel 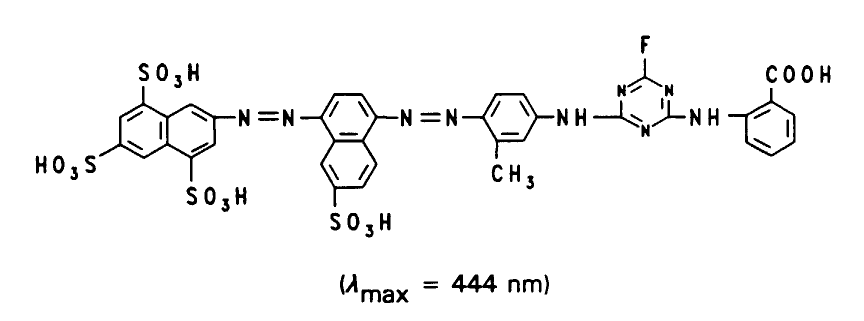 kann in üblicher Weise durch Aussalzen mittels Natriumchlorid oder durch Sprühtrocknung des Filtrats als Alkalimetallsalz (Natriumssalz) isoliert werden.

Die erfindungsgemäße Disazoverbindung besitzt sehr gute faserreaktive Farbstoffeigenschaften und liefert nach den in der Technik üblichen Applikations- und Fixierverfahren auf den in der Beschreibung genannten Materialien, wie insbesondere Cellulosefasermaterialien, farbstarke, rotstichig braune Färbungen und Drucke mit guten Echtheitseigenschaften, von denen insbesondere die gute Lichtechtheit und die gute Hypochloritechtheit hervorgehoben werden können.

### Beispiel 3

253 Teile Anilin-2,5-disulfonsäure werden in üblicher Weise in 1000 Teilen verdünnter Schwefelsäure diazotiert; anschließend werden 223 Teile 1-Aminonaphthalin-6-sulfonsäure hinzugegeben und die Kupplungsreaktion bei einem pH-Wert von 4 bis 5 und einer Temperatur von 10 bis 15°C durchgeführt. Danach wird die erhaltene Amino-Azoverbindung indirekt diazotiert und überschüssiges Nitrit mit Amidosulfonsäure zerstört. Eine Lösung von 108 Teilen 3-Methyl-anilin in 100 Teilen einer 33%igen wäßrigen Salzsäure werden hinzugegeben, der pH-Wert mit Natriumcarbonat auf 3,5 bis 4 gestellt und die Kupplungsreaktion bei etwa 20°C durchgeführt. Anschließend wird dem Ansatz das Reaktionsprodukt aus 135 Teilen Cyanurfluorid, 139 Teilen Anthranilsäure und 84 Teilen Natriumfluorid bei 0°C und einem pH-Wert von unterhalb 1 zugegeben; man rührt noch 1 Stunde bei 20°C unter Einhaltung des pH-Wertes nach.

Die erhaltene Lösung des erfindungsgemäßen Disazofarbstoffes, der, in Form der freien Säure geschrieben, die Formel 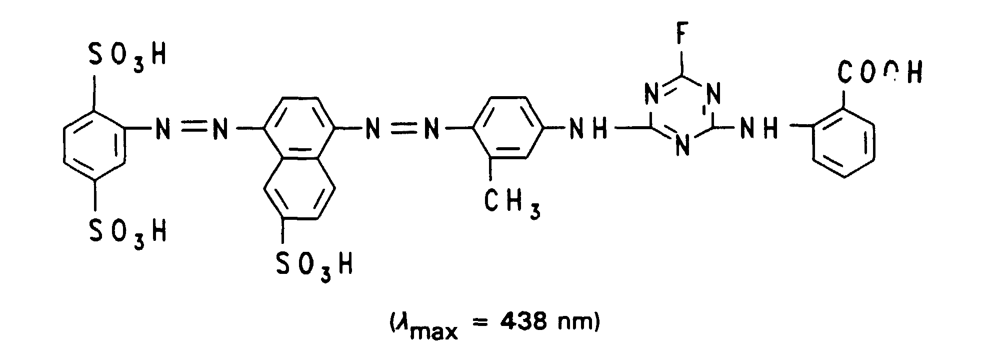 besitzt, wird mittels 20 Teilen Kieselgur und Filtration geklärt. Der Farbstoff wird durch Aussalzen mit Natriumchlorid aus dem Filtrat oder durch Sprühtrocknung des Filtrates isoliert. Er besitzt gute Farbstoffeigenschaften und man erhält mit ihm beispielsweise auf Baumwolle farbstarke, rotstichig braune Färbungen und Drucke mit guten Echtheitseigenschaften.

## Patentansprüche

1. Disazoverbindung der allgemeinen Formel (1) 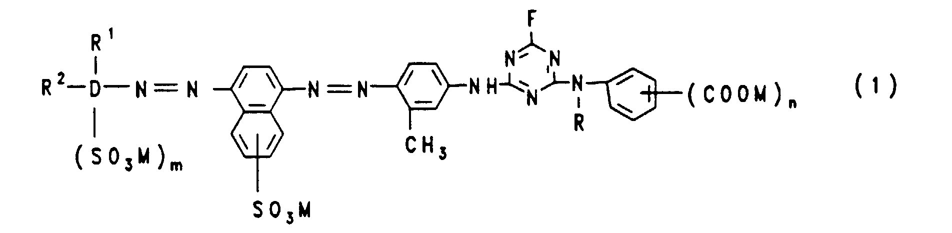 in welcher bedeutet:
D ist ein Phenyl- oder Naphthylrest;
R¹ ist Wasserstoff, Alkyl von 1 bis 4 C-Atomen, Alkoxy von 1 bis 4 C-Atomen, Carboxy, Halogen, Alkanoylamino von 2 bis 5 C-Atomen oder Benzoylamino;
R² ist Wasserstoff oder Alkyl von 1 bis 4 C-Atomen;
M ist Wasserstoff oder ein Alkalimetall oder ein anderes salzbildendes Metall;
m ist die Zahl 1, 2 oder 3;
n ist die Zahl 1, wobei die Gruppe -COOM in ortho-Stellung zur Gruppe -N(R)- an den Benzolkern gebunden ist;
R ist Wasserstoff oder Alkyl von 1 bis 4 C-Atomen;
die Gruppe -SO₃M an der Naphthalin-Mittelkomponente ist in 6-, 7- oder 8-Stellung an diesen Naphth-4,1-ylen-Rest gebunden.

2. Disazoverbindung nach Anspruch 1, dadurch gekennzeichnet, daß R Wasserstoff ist.

3. Disazoverbindung nach mindestens einem der Ansprüche 1 oder 2, dadurch gekennzeichnet, daß D der Phenylrest ist, R¹ Wasserstoff, Methyl, Methoxy, Carboxy, Chlor, Brom, Acetylamino oder Benzoylamino bedeutet, R² Wasserstoff oder Methyl ist und m für die Zahl 1 oder 2 steht.

4. Disazoverbindung nach mindestens einem der Ansprüche 1 oder 2, dadurch gekennzeichnet, daß D der 2-Naphthyl-Rest ist, R¹ und R² beide Wasserstoff bedeuten und m für die Zahl 2 oder 3 steht.

5. Disazoverbindung nach mindestens einem der Ansprüche 1 oder 2, dadurch gekennzeichnet, daß D Phenyl ist und R¹ und R² beide Wasserstoff bedeuten.

6. Disazoverbindung nach Anspruch 5, dadurch gekennzeichnet, daß m die Zahl 2 ist.

7. Verfahren zur Herstellung einer Disazoverbindung von Anspruch 1, dadurch gekennzeichnet, daß man eine Difluor-triazinylamino-Disazoverbindung der allgemeinen Formel (3) 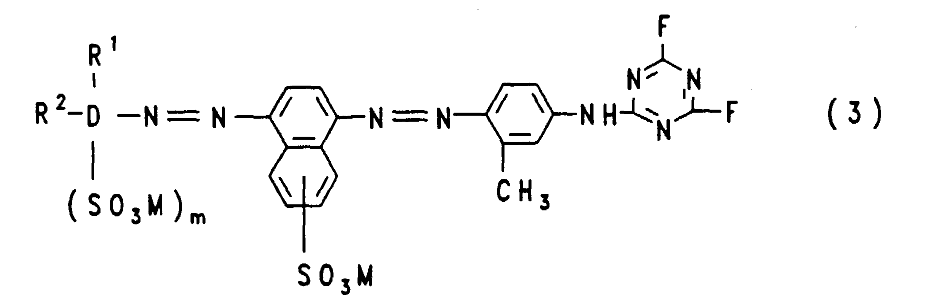 in welcher D, R¹, R², M und m eine der in Anspruch 1 genannten Bedeutungen besitzen, mit einer Verbindung der allgemeinen Formel (4) 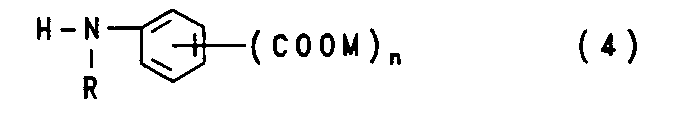 in welcher R, M und n eine der in Anspruch 1 genannten Bedeutungen haben, umsetzt,
oder daß man eine Verbindung der allgemeinen Formel (2) 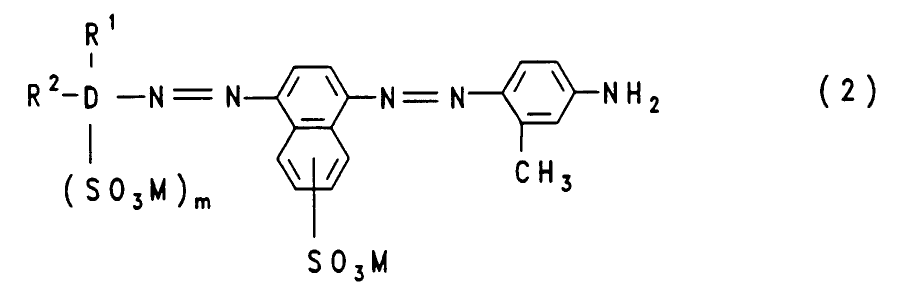 in welcher D, R¹, R², M und m die in Anspruch 1 genannten Bedeutungen haben, mit einer Verbindung der allgemeinen Formel (5) 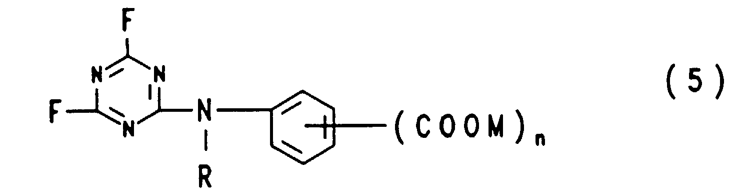 in welcher R, M und n eine der in Anspruch 1 genannten Bedeutungen haben, umsetzt.

8. Verwendung eines Farbstoffes von mindestens einem der Ansprüche 1 bis 6 oder eines nach Anspruch 7 hergestellten Farbstoffes zum Färben und Bedrucken von hydroxy- und/oder carbonamidgruppenhaltigem Material, insbesondere Fasermaterial.

9. Verfahren zum Färben und Bedrucken von hydroxy- und/oder carbonamidgruppenhaltigem Material, vorzugsweise Fasermaterial, bei welchem man einen Farbstoff auf das Material aufbringt und den Farbstoff auf dem Mateial mittels Wärme oder mit Hilfe eines alkalisch wirkenden Mittels oder mittels Wärme und mit Hilfe eines alkalisch wirkenden Mittels fixiert, dadurch gekennzeichnet, daß der Farbstoff ein Farbstoff von mindestens einem der Ansprüche 1 bis 6 oder ein nach Anspruch 7 hergestellter Farbstoff ist.

## Claims

1. A disazo compound of the formula (1) 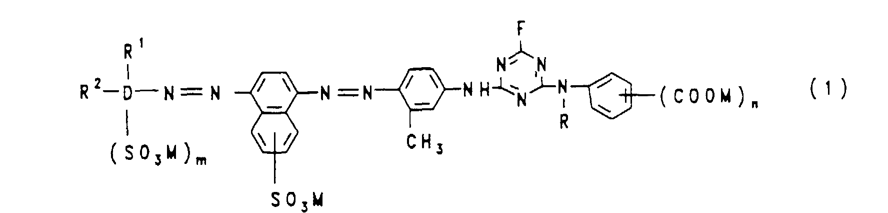 where
D is a phenyl or naphthyl radical,
R¹ is hydrogen, alkyl of 1 to 4 carbon atoms, alkoxy of 1 to 4 carbon atoms, carboxyl, halogen, alkanoylamino of 2 to 5 carbon atoms or benzoylamino,
R² is hydrogen or alkyl of 1 to 4 carbon atoms,
M is hydrogen or an alkali metal or another salt-forming metal,
m is 1, 2 or 3,
n is 1, the group -COOM being bonded to the benzene ring ortho to the group -N(R)-,
R is hydrogen or alkyl of 1 to 4 carbon atoms,
the group -SO₃M on the naphthalene middle component is attached to this naphth-4,1-ylene radical in position 6, 7 or 8.

2. A disazo compound as claimed in claim 1, wherein R is hydrogen.

3. A disazo compound as claimed in at least one of claims 1 or 2, wherein D is phenyl, R¹ is hydrogen, methyl, methoxy, carboxyl, chlorine, bromine, acetylamino or benzoylamino, R² is hydrogen or methyl, and m is 1 or 2.

4. A disazo compound as claimed in at least one of claims 1 or 2, wherein D is 2-naphthyl, R¹ and R² are each hydrogen, and m is 2 or 3.

5. A disazo compound as claimed in at least one of claims 1 or 2, wherein D is phenyl and R¹ and R² are each hydrogen.

6. A disazo compound as claimed in claim 5, wherein m is 2.

7. A process for preparing a disazo compound of claim 1, which comprises reacting a difluorotriazinylaminodisazo compound of the formula (3) 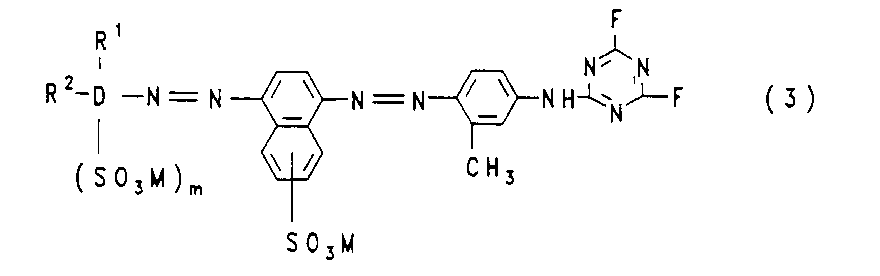 where D, R¹, R², M and m are each as defined in claim 1, with a compound of the formula (4) 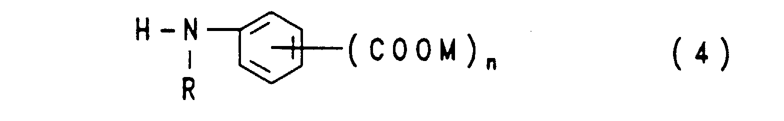 where R, M and n are each as defined in claim 1, or reacting a compound of the formula (2) 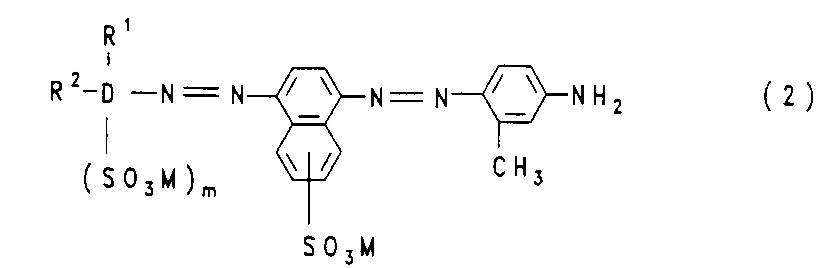 where D, R¹, R², M and m are each as defined in claim 1, with a compound of the formula (5) 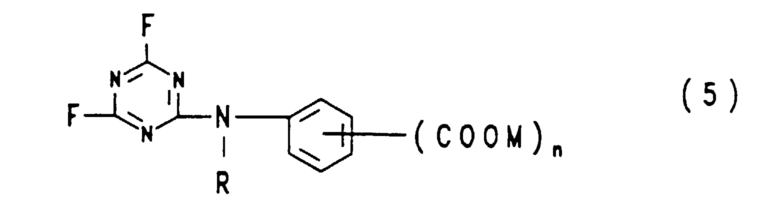 where R, M and n are each as defined in claim 1.

8. The use of a dye of at least one of claims 1 to 6 or of a dye prepared as claimed in claim 7 for dyeing and printing hydroxyl- and/or carboxamido-containing material, in particular fiber material.

9. A process for dyeing and printing hydroxyl- and/or carboxamido-containing material, preferably fiber material, by applying a dye to the material and fixing it thereon by means of heat or with the aid of an alkali or by means of heat and with the aid of an alkali, which comprises using a dye as set forth in at least one of claims 1 to 6 or as prepared as claimed in claim 7.

## Revendications

1. Composé disazo de formule générale (1) 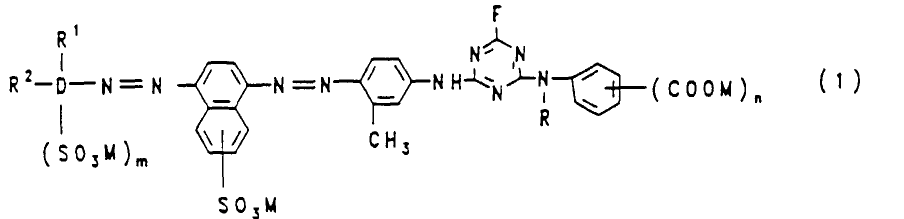 dans lequel les symboles :
D est un radical phényle ou naphtyle ;
R¹ est l'hydrogène, un groupement alkyle de 1 à 4 atomes de C, alcoxy de 1 à 4 atomes de C, carboxyle, halogène, alkanoylamino de 2 à 5 atomes de C ou benzoylamino ;
R² est l'hydrogène ou un groupement alkyle de 1 à 4 atomes de C ;
M est l'hydrogène ou un métal alcalin ou un autre métal pouvant former des sels ;
m est le nombre 1, 2 ou 3 ;
n est le nombre 1, le groupement -COOM étant lié au noyau benzénique en position ortho par rapport au groupe -N(R)- ;
R est l'hydrogène ou un groupement alkyle de 1 à 4 atomes de C ; le groupement -SO₃M situé sur le composant naphtalène central est lié à ce radical napht-4,1-ylène en position 6, 7 ou 8.

2. Composé disazo selon la revendication 1, caractérisé en ce que R est l'hydrogène.

3. Composé disazo selon au moins l'une des revendications 1 ou 2, caractérisé en ce que D est le radical phényle, R¹ représente l'hydrogène, un groupement méthyle, méthoxy, carboxyle, le chlore, le brome, un groupement acétylamino ou benzoylamino ; R² est l'hydrogène ou un groupement méthyle, et m représente le nombre 1 ou 2.

4. Composé disazo selon au moins l'une des revendications 1 ou 2, caractérisé en ce que D est le radical 2-naphtyle, R¹ et R² représentent tous deux l'hydrogène, et m représente le nombre 2 ou 3.

5. Composé disazo selon au moins l'une des revendications 1 ou 2, caractérisé en ce que D est le radical phényle, et R¹ et R² représentent tous deux l'hydrogène.

6. Composé disazo selon la revendication 5, caractérisé en ce que m est le nombre 2.

7. Procédé pour la fabrication d'un composé disazo de la revendication 1, caractérisé en ce que l'on fait réagir un composé difluoro-triazinylamino-disazo de formule générale (3) 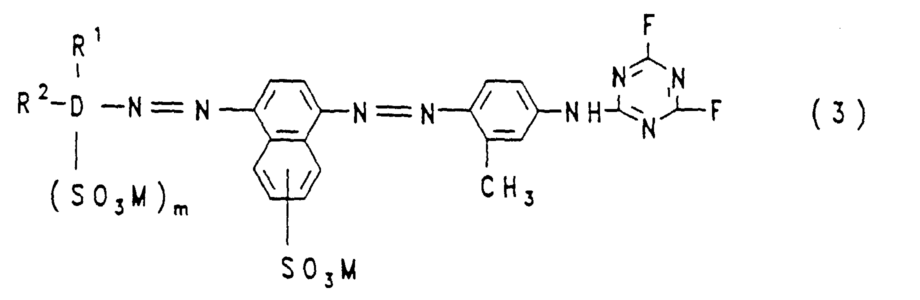 dans laquelle D, R¹, R², M et m ont l'une des significations mentionnées dans la revendication 1, avec un composé de formule générale (4) 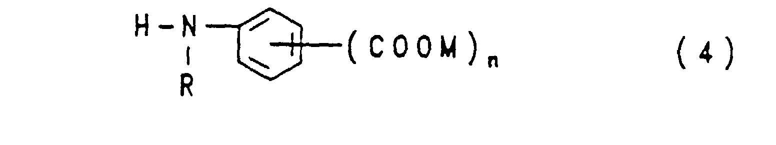 dans laquelle R, M et n ont l'une des significations mentionnées dans la revendication 1,
ou en ce que l'on fait réagir un composé de formule générale (2) 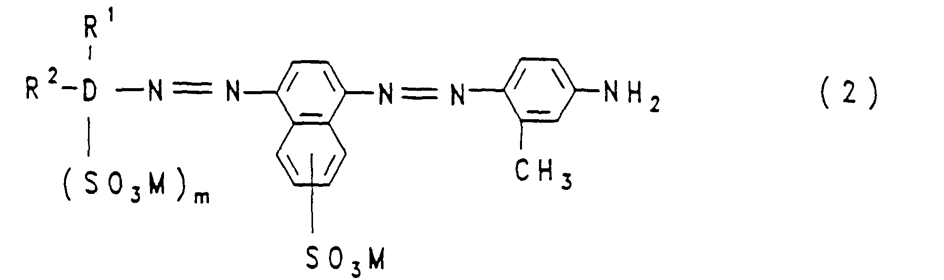 dans laquelle D, R¹, R², M et m ont les significations mentionnées dans la revendication 1, avec un composé de formule générale (5) 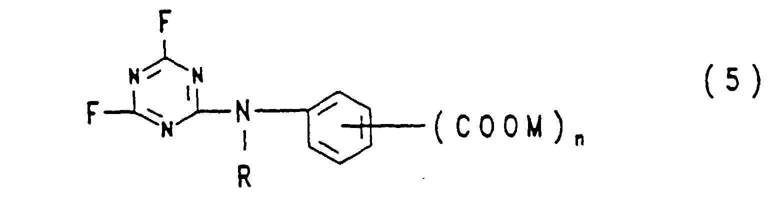 dans laquelle R, M et n ont l'une des significations mentionnées dans la revendication 1.

8. Utilisation d'un colorant d'au moins l'une des revendications 1 à 6, ou d'un colorant préparé selon la revendication 7, pour colorer et imprimer un matériau contenant des groupements hydroxyle et/ou carbonamide, en particulier un matériau fibreux.

9. Procédé pour colorer et imprimer un matériau contenant des groupements hydroxyle et/ou carbonamide, de préférence un matériau fibreux, dans lequel on applique un colorant sur le matériau et l'on fixe le colorant sur le matériau au moyen de chaleur ou à l'aide d'un milieu à effet alcalin, ou au moyen de chaleur et à l'aide d'un milieu à effet alcalin, caractérisé en ce que le colorant est un colorant d'au moins l'une des revendications 1 à 6, ou un colorant préparé selon la revendication 7.
